(19) Europäisches Patentamt
European Patent Office
Office européen des brevets

(11) Veröffentlichungsnummer: **0 086 278**
**B1**

(12) # EUROPÄISCHE PATENTSCHRIFT

(45) Veröffentlichungstag der Patentschrift: **26.02.86**

(21) Anmeldenummer: **82112106.8**

(22) Anmeldetag: **30.12.82**

(51) Int. Cl.⁴: **C 07 D 233/78**, C 07 D 233/86, C 08 G 18/67, C 08 G 73/06, C 08 J 5/18, C 09 D 3/49

(54) **Verfahren zur Herstellung von Hydantoinestern.**

(30) Priorität: **13.01.82 DE 3200704**

(43) Veröffentlichungstag der Anmeldung: **24.08.83 Patentblatt 83/34**

(45) Bekanntmachung des Hinweises auf die Patenterteilung: **26.02.86 Patentblatt 86/9**

(84) Benannte Vertragsstaaten: **AT DE FR GB IT**

(56) Entgegenhaltungen:
**EP-A-0 002 444**
**EP-A-0 033 477**
**DE-A-2 714 655**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Zecher, Wilfried, Dr., Treptower Strasse 6, D-5090 Leverkusen 1 (DE)**
Erfinder: **Merten, Rudolf, Dr., Berta-von-Suttner-Strasse 55, D-5090 Leverkusen (DE)**

**EP 0 086 278 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Es ist bekannt, daß man Hydantoine durch Umsetzung von α-Amino-carbonsäure-derivaten mit Isocyanaten erhält (Am. Chem. J. *45*, 382 [1911]). Weitere Herstellungswege sind beispielsweise die Umsetzung von α-Amino-nitrilen mit Isocyanaten und nachfolgende Verseifung der gebildeten Imino-Verbindungen oder die Kondensation von α,β-ungesättigten Carbonsäurederivaten mit Isocyanaten (DE—OS—3 003 773, bzw. EP—A—0 033 477 und EP—A—0 002 444).

Monomolekulare Hydrantoine können im Pharmabereich und auf dem Pflanzenschutzsektor verwendet werden. Polyhydantoine eignen sich z.B. als temperaturbeständige Kunststoffe, beispielsweise auf dem Elektroisoliersektor (FR—A—1 484 694).

Die Erfindung betrifft ein Verfahren zur Herstellung von Hydantoinestern, das dadurch gekennzeichnet ist, daß organische Isocyanate mit Ethen-1,2-dicarbonsäureamidestern der Formeln (Ia—c)

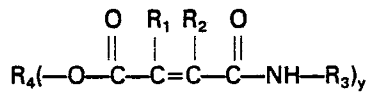
.  (Ib)

oder

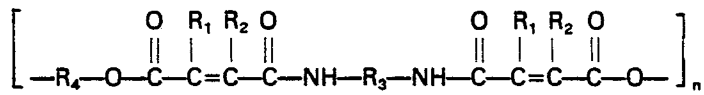
(Ic)

in denen

$R^1$, $R^2$, $R^3$ und $R^4$ unabhängig voneinander für einen aliphatischen, cycloaliphatischen, aliphatischen-aromatischen oder aromatischen Rest stehen und

$R^1$, $R^2$ und $R^3$ außerdem noch für Wasserstoff und

$R^1$ und $R^2$ für Halogen stehen können,

x und y ganze Zahlen von 1 bis 3 bedeuten und

n eine ganze Zahl von 1 bis 200 bedeutet bei einer Temperatur von 50 bis 250°C, umgesetzt werden.

Die Ethen-1,2-dicarbonsäureamidester können in der cis- und/oder trans-Form vorliegen.

Der Verlauf der Umsetzung ist überraschend, da bei Amidestern dieser Konstitution die Cyclisierung unter Abspaltung von Alkohol zu Imiden und deren weitere Reaktion mit Isocyanaten zu erwarten wären. Der glatte Verlauf und die hohen Ausbeuten der erfindungsgemäßen Umsetzung ermöglichen auch den Aufbau von Polymeren. Im Gegensatz zu den bekannten Verfahren zur Herstellung von Hydrantoinen verläuft die Umsetzung ohne Bildung von Spaltprodukten wie Kohlendioxid und Alkoholen, so daß Nebenreaktionen, die zum Kettenabbruch führen, oder Blasenbildung in den Polymeren vermieden werden können.

Die erfindungsgemäß als Ausgangsmaterialien verwendeten Ethen-1,2-dicarbonsäureamidester sind bekannt (Beilstein *12*, 305). Sie können z.B. aus ungesättigten Amidsäuren durch Veresterung hergestellt werden. Sie können in Substanz eingesetzt oder auch in situ im Umsetzungsgemisch aus den Komponenten hergestellt werden.

Im erfindungsgemäßen Verfahren werden vor allem Ethen-1,2-dicarbonsäureamidester der Formeln (Ia—c) eingesetzt, in denen

$R^1$, $R^2$, $R^3$ und $R^4$ unabhängig voneiander für einen aliphatischen Rest mit 1 bis 20 C-Atomen, cycloaliphatischen Rest mit 5 bis 10 C-Atomen, einen aliphatisch-aromatischen Rest mit 7 bis 15 C-Atomen oder einen aromatischen Rest mit 6 bis 16 C-Atomen stehen,

$R^1$, $R^2$ und $R^3$ außerdem für Wasserstoff stehen können und

$R^1$ und $R^2$ gleich oder verschieden für Fluor, Chlor und Brom stehen und

x und y für eine ganze Zahl von 1 bis 3 steht und

n eine ganze Zahl von 5 bis 50 bedeutet.

Bevorzugt sind Ethen-1,2-dicarbonsäureamidester der Formeln (Ia—c), in denen

$R^1$ und $R^2$ für Wasserstoff,

$R^3$ für einen aliphatischen Rest mit 1 bis 20 C-Atomen, aliphatisch-aromatischen Rest 7 bis 15 C-Atomen oder aromatischen Rest mit 6 bis 16 C-Atomen steht und

$R^4$ für einen aliphatischen Rest mit 1 bis 20 C-Atomen oder für einen aliphatisch-aromatischen Rest mit 7 bis 15 C-Atomen steht.

Die Reste $R^1$ und $R^2$ leiten sich beispielsweise von Wasserstoff, Fluor, Chlor, Brom, Methan, Ethan, Hexan, Cyclohexan, Propen, Toluol und Benzol ab und können weiterhin zusammen einen Ring mit bis zu acht Kohlenstoffatomen bilden.

Die Reste $R^3$ und $R^4$ leiten sich beispielsweise von Methan, Ethan, n-, iso- und tert.-Butan, Hexan, Eicosan, Propen, Butin, Cyclohexan, Benzol, Naphthalin, Diphenylmethan, Diphenylether, Diphenylsulfon, ω- oder kernsubstituiertem Toluol, Xylol, Polyethern, Polyestern, Polyharnstoffen und Polyurethanen ab. Sie können einfach oder mehrfach z.B. mit Halogen oder Alkyl-, Carbonsäure-, Carbonester-, Hydroxy- und Amino-Gruppen substituiert sein.

Als Monoisocyanate im Sinne der Erfindung werden aliphatische und aromatische, gegebenenfalls durch Heteroatome substituierte Verbindungen mit einer NCO-Gruppe im Molekül eingesetzt, z.B. Alkylisocyanate wie Ethyl-, Methyl-, Butyl-, Dodecyl- und Stearylisocyanat, aromatische, gegebenenfalls substituierte Monoisocyanate wie Phenyl-, Tolyl-, Isopropyl-, Nonylisocyanat, Nitro-, Alkoxy-, Aroxy-, Chlor-, Dichlor-, Trichlor-, Tetra-, Pentachlor-, Benzyl-, Brom-phenyl-isocyanat oder Isocyanatobenzoesäureester, Phthalsäureester, Isophthalsäureester, Isocyanatobenzonitril, cycloaliphatische Isocyanate wie Cyclohexylisocyanat und ungesättigte Isocyanate wie Allyl-, Oleyl-, Cyclohexenyl-isocyanat.

Als erfindungsgemäß einzusetzende Ausgangskomponenten kommen weiterhin aliphatische, cycloaliphatische, araliphatische, aromatische und heterocyclische Polyisocyanate, vorzugsweise Diisocyanate, in Betracht, (vgl. Annalen, 562, Seite 75 bis 136), beispielsweise Ethylendiisocyanat, 1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanato-methyl-cyclohexan (DE—A—1 202 785), 2,4- und 2,6-Hexahydrotoluylendiisocyanat sowie beliebige Gemische dieser Isomeren, Hexahydro-1,3- und/oder -1,4-phenylen-diisocyanat, Perhydro-2,4' und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder 4,4'-diisocyanat, Naphthylen-1,5-diisocyanat, Triphenylmethan-4,4',4''-Triisocyanat, Polyphenyl-polymethylen-polyisocyanate wie sie durch Anilin-Formaldehyd-Kondensation und anschließende Phosgenierung erhalten werden (z.B. GB—A—874 430 und 848 671), perchlorierte Arylpolyisocyanate (z.B. DE—A—1 157 601), Carbodiimidgruppen aufweisende Polyisocyanate (z.B. DE—B—1 092 007), Diisocyanate (z.B. US—A—3 492 330), Allophanatgruppen aufweisende Polyisocyanate (z.B. GB—A—99 890, BE—B—761 626 und BL—PA—7 102 524), Isocyanuratgruppen aufweisende Polyisocyanate (z.B. DE—B—1 022 789, 1 222 067 und 1 027 394, DE—A—1 929 034 und 2 004 048), Urethangruppen aufweisende Polyisocyanate (z.B. BE—B—752 261 oder US—A—3 394 164), acylierte Harnstoffgruppen aufweisende Polyisocyanate (z.B. DE—B—1 230 778), Biuretgruppen aufweisende Polyisocyanate (z.B. DE—B—1 101 394, GB—A—889 050 und FR—B—1 017 514), durch Telomerisationsreaktionen hergestellte Polyisocyanate (z.B. BE—B—723 640), Estergruppen aufweisende Polyisocyanate (z.B. GB—A—956 474 und 1 072 956, US—A—3 567 763 und DE—B—1 231 688) und Umsetzungsprodukte der oben genannten Isocyanate mit Acetylen (gemäß DE—B—1 072 358).

Es ist auch möglich, die bei der technischen Isocyanatherstellung anfallenden Isocyanatgruppen aufweisenden Destillationsrückstände, gegebenenfalls gelöst in einem oder mehreren der vorgenannten Polyisocyanate, einzusetzen. Ferner ist es möglich, belieibige Mischungen der vorgenannten Polyisocyanate zu verwenden.

Vorzugsweise eignen sich Mono- bzw. Polyiso(thio)cyanate der allgemeinen Formeln (II)

$$R^5(\text{—NCO})_z \text{ bzw. } R^5(\text{—NCS})_z \qquad (II),$$

in denen

$R^5$ für einen, gegebenenfalls mit Halogen, Alkyl- und/oder Arylgruppen substituierten aliphatischen Rest mit 1—20 C-Atomen, einen aromatischen Rest mit 5—12 C-Atomen, einen cycloaliphatischen Rest mit 5—12 C-Atomen, einen aliphatischen-aromatischen Rest mit 6—20 C-Atomen und einen Heteroatome wie N, O oder S enthaltenden aromatischen oder cycloaliphatischen Rest mit 5—12 C-Atomen und

z für eine ganze Zahl von 1—4 steht.

Besonders bevorzugt sind aliphatische Reste mit 2—12 C-Atomen, Arylreste wie Phenyl, Tolyl, Naphthyl, Diphenylmethan und Diphenyletherreste.

z steht vorzugsweise für eine ganze Zahl von 1—3, besonders bevorzugt für die Zahl 2.

Bevorzugt verwendet werden die technisch leicht zugänglichen Gemische aus Toluylen-diisocyanaten, m-Phenylendiisocyanat, Phenylisocyanat und seine Substitutionsprodukte,-Methylisocyanat, sowie phosgenierte Kondensate aus Anilin und Formaldehyd mit Polyphenylenmethylen-struktur und die symmetrischen Verbindungen 4,4'-Diisocyanato-di-phenylmethan, 4,4'-Diisocyanato-diphenylether, p-Phenylendiisocyanat, 4,4'-Diisocyanato-diphenylmethan, analoge hydroaromatische Diisocyanate, sowie aliphatische Diisocyanate mit 2—12 C-Atomen wie Hexamethylendiisocyanat und von Isophoron abgeleitete Diisocyanate.

Die Isocyanate können in freier Form eingesetzt werden. Weiterhin ist as möglich sie zum Teil oder vollständig auch in Form ihrer, beim Umsatz mit reaktiven Wasserstoff enthaltenden Verbindungen zugänglichen, und unter den Reaktionsbedingungen als Abspalter reagierenden Derivate, einzusetzen.

Vorzugsweise werden als Abspalter die aus Lactamen wie Caprolactam und Pyrrolidon zugänglichen Acyl-Harnstoffe und die aus aromatischen und aliphatischen Mono- und Polyhydroxy-Verbindungen erhaltenen Carbamidsäureester eingesetzt, die z.B. den allgemeinen Formeln

$$R^5(\text{—NH—}\underset{\underset{\textstyle O}{\|}}{C}\text{—O—A})_2 \text{ bzw. } \left[\text{—}\underset{\underset{\textstyle O}{\|}}{C}\text{—NH—}R^5\text{—NH—}\underset{\underset{\textstyle O}{\|}}{C}\text{—O—B—O—}\right]_n$$

in denen

$R^5$ und z die oben angegebene Bedeutung haben und

A der organische Rest einer Monohydroxyverbindung und

B der organische Rest einer bis- oder trisfunktionellen Hydroxyverbindung, vorzugsweise ein aliphatischer Rest mit 1—10 C-Atomen, einen cycloaliphatischen Rest mit 5—10 C-Atomen, ein aliphatischer-aromatischer Rest mit 7—12 C-Atomen und ein aromatischer Rest mit 5—12 C-Atomen ist, wobei diese auch mit Alkyl- und/oder Arylgruppen substituiert sein können, und

n für eine ganze Zahl von 1—1000, vorzugsweise 1—100 steht, entsprechen.

Als Beispiele seien die Carbamidsäureester aus Phenol, isomeren Kresolen, deren technischen Gemischen und ähnlichen aromatischen Hydroxylverbindungen, aliphatische Monoalkohole wie Methanol, Ethanol, Propanol, Isopropanol, Butanol, Isobutanol, Diethylenglykolmonomethylester, Cyclohexanol, Benzylalkohol und aliphatische Di- oder Polyole wie Ethylenglykol und Trimethylolpropan aufgeführt.

Die Urethane können als solche eingesetzt oder erst in situ durch Umsetzung mit Alkoholen erzeugt werden.

Anstelle der genannten (Poly)Isocyanate können auch die analogen (Poly)Isothiocyanate eingesetzt werden.

Die erfindungsgemäße Umsetzung soll beispielhaft durch das nachfolgende Schema für monomolekulare Hydantoine erläutert werden:

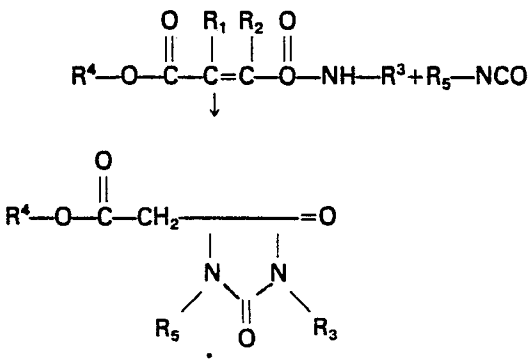

wobei die Reste $R^1$ bis $R^5$ die bei Formel (I) angegebene Bedeutung haben. Entsprechend werden für x, y, n und/oder z>1 in den Formeln (I) und (II) oligeomere, polymere oder vernetzte Polyhydantoine erhalten.

Die erfindungsgemäßen Hydantoine können eindeutig durch IR-Spektren, in denen die charakteristischen Banden für Hydantoine und Ester auftreten, identifiziert werden. Die höhermolekularen Hydantoine zeigen eine Lösungsviskosität von 200 bis 200 000 zu mPas, vorzugsweise von 1000 bis 50 000, die z.B. an einer 30 Gew.%igen Lösung in Kresol oder Butyrolacton bei 25°C bestimmt wird.

Die erfindungsgemäße Umsetzung kann in Lösungsmitteln, die unter den Umsetzungsbedingungen nicht reagieren oder nur lockere Additionsverbindungen bilden, oder auch in einem Überschuß einer der Umsetzungskomponenten ausgeführt werden. Geeignete Lösungsmittel sind beispielsweise (Halogen)-Kohlenwasserstoffe, Phenole, Alkohole, Ester, Lactone, Ketone, Ether, substituierte Amide, Nitrile, Phosphorsäureamide, Sulfoxide und Sulfone, beispielsweise Xylole, o-Dichlorbenzol, Phenol, Kresol, Benzoesäurealkylester, Butyrolacton, Caprolacton, Acetophenon, Cyclohexanon, Benzylalkohol, Ethylenglykol, Glykolmonomethyletheracetat, Diethylenglykolmonomethylether, Diethylenglykoldimethylether, Dimethylformamid, N-Methylpyrrolidon, Caprolactam, Benzonitril, Hexamethylphosphorsäuretriamid, Dimethylsulfoxid, Tetramethylensulfon.

Bei der Durchführung der erfindungsgemäßen Umsetzung können Lösungsmittelgemische verwendet werden.

Die Durchführung der erfindungsgemäßen Umsetzung kann in der Weise erfolgen, daß die Komponenten mit oder ohne Lösungsmittel einige Minuten bis zu mehreren Stunden bei Temperaturen von etwa 50—250°C, gehalten werden. Der Verlauf der Umsetzung kann IR-spektroskopisch verfolgt werden.

Es kann vorteilhaft sein, die Umsetzung in mehreren Stufen durchzuführen oder die einzelnen Komponenten in unterschiedlicher Reihenfolge oder bei verschiedenen Temperaturen zusammenzugeben. Insbesondere bei der Herstellung von Polymeren kann in einer ersten Stufe, z.B. in einem Lösungsmittel, ein Kondensationsprodukt hergestellt werden. Dieses kann bei höheren Temperaturen unter Kettenverlängerung oder Vernetzung und gegebenenfalls unter Verdampfen des Lösungsmittels in ein hochmolekulares Produkt, z.B. einen Lackfilm übergeführt werden.

Im allgemeinen werden pro Val Esteramid 1 Val Isocyanat eingesetzt. Doch sind sehr weitgehende Abweischungen von diesen Mengenverhältnissen möglich.

Aus Monoamidestern und monofunktionellen Isocyanaten werden monomolekulare Hydantoine, aus Oligoamidestern und Diisocyanaten in Abhängigkeit von den stöchiometrischen Verhältnissen höhermolekulare Polyhydantoine oder Oligomere, z.B. Hydatoinisocyanate, erhalten.

Bevorzugt wird die erfindungsgemäße Umsetzung in Gegenwart von Katalysatoren ausgeführt. Es können beispielsweise Amine wie Triethylamin, 1,4-Diazabicyclo-(2,2,2)octan, N-Ethyl-morpholin und N-methyl-imidazol, organische und anorganische Metallverbindungen, insbesondere Eisen-, Blei- Zink-, Zinn-, Kupfer-, Kobalt- und Titan-Verbindungen, z.B. Eisen (III)-chlorid, Kobaltacetat, Bleioxid, Bleiacetat, Zinnoctoat, Dibutyl-zinndilaurat, Kupfer-acetylacetonat, Titantetrabutylat, Alkali-phenolate und Natriumcyanid, Phosphorverbindungen wie Trialkylphosphin und Methylphospholinoxid, Hydroxy-Aromaten wie Phenol, Hydrochinon und Resorcin eingesetzt werden.

Bevorzugt werden Amine, besonders bevorzugt Triethylendiamin und Bis-dimethylaminoethyl-methylamin eingesetzt.

Außer den eingangs erwähnten Verwendungsmöglichkeiten können die nach dem erfindungsgemäßen Verfahren herstellbaren Polyhydantoine als Kleber, Lacke, Folien und Formkörper verwendet werden. Sie zeichnen sich durch besondere Temperaturbeständigkeit aus. Ihre Eigenschaften können für die verschiedenen Einsatzgebiete durch Zusatz von Füllstoffen, Pigmenten, nieder- und hochmolekularen Komponenten, z.B. zur Herstellung von Lacken und Folien durch Abmischen mit Polyestern und Polycarbamidestern in weiten Grenzen variiert werden (DE—A—2 654 112).

Beispiel 1

In 150 g Xylol werden 32,9 g N-Phenylfumaramidsäureethylester und 17,9 g Phenylisocyanat mit 0,5 g Triethylendiamin als Katalysator jeweils 2 Stdn. auf 80, 100, 120 und 140°C erhitzt. Anschließend wird das Lösungsmittel in einem Rotationsverdampfer unter Vakuum abgezogen und der harzige Rückstand aus 50 g Ethanol umkristallisiert. Man erhält 45 g des 1,3-Diphenyl-5-(ethoxycarbonyl-methyl)-hydantoins in farblosen Polyedern vom Schmelzpunkt 84—85°C mit den für Hydantoine charakteristischen Banden im IR-Spektrum bei 1720 und 1770 $cm^{-1}$.

$C_{19}H_{18}N_2O_4$(338)

| | C | H | N |
|---|---|---|---|
| ber. | 67,5 | 5,3 | 8,3% |
| gef. | 67,6 | 5,6 | 8,5% |

Beispiel 2

In 90 g Butyrolacton werden 28 g N-Phenyl-maleinamidsäuremethylester und 16,2 g Phenylisocyanat erhitzt. Die Temperatur wird, bei 80°C beginnend, in jeweils einer Stunde um 10°C gesteigert, bis 160°C erreicht werden und dann noch 2 Stdn. in diesem Bereich gehalten. Nach dem Erkalten wird das Reaktionsgemisch mit Wasser versetzt. Man erhält eine braune Fällung, die bei der Umkristallisation aus Methanol das reine 1,3-Diphenyl-5-(methoxycarboxylmethyl)-hydantoin in farblosen Polyedern vom Schmelzpunkt 146—147°C ergibt.

$C_{18}H_{16}N_2O_4$(324)

| | C | H | N |
|---|---|---|---|
| ber. | 66,7 | 4,9 | 8,6% |
| gef. | 66,6 | 4,9 | 8,8% |

Beispiel 3

Entsprechend Beispiel 2 werden in 65 g Butyrolacton 20,6 g N-Phenyl-fumaramidsäuremethylester und 12 g Phenylisocyanat umgesetzt. Man erhält bei der Umkristallisation aus Methanol 20 g 1,3-Diphenyl-5-(methoxy-carbonyl-methyl)-hydantoin vom Schmelzpunkt 146—147°.

$C_{18}H_{16}N_2O_4$(324)

| | C | H | N |
|---|---|---|---|
| ber. | 66,7 | 4,9 | 8,6% |
| gef. | 66,5 | 4,9 | 8,7% |

Beispiel 4

105,5 g 4,4'-Bis-(fumarsäuremethylester-amido)-diphenylmethan, 6,5 g 4,4'-Diisocyanato-diphenylmethan und 0,5 g Triethylendiamin werden in 340 g eines technischen Kresolgemisches eingetragen und 5 Stdn. bei 180°C gerührt. Der Polyhydantoin-(5)-essigsäuremethylester wird als hellbraune Lösung mit der Viskosität $\eta^{25}$=7500 mPas, einem Festgehalt von 33% und Hydantoin-Banden bei 1725 und 1780 $cm^{-1}$ erhalten.

Eine Probe der Lacklösung wird auf ein Prüfblech aufgestrichen und bei 200°C und dann bei 300°C zu einem klaren elastischen Lackfilm gebrannt.

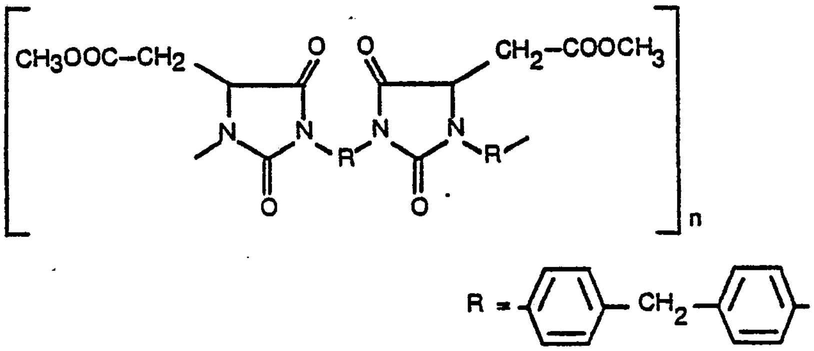

Beispiel 5

In 346 g Butyrolacton werden 108 g 4,4'-Bis-(maleinsäuremethylester-amido)-diphenylmethan und 67,6 g 4,4'-Diisocyanato-diphenylmethan, jeweils 2 Stdn. bei 80, 120, 140 und 160° gerührt. Man erhält den Poly-hydantoin-(5)-essigsäuremethylester als braune viskose Lösung mit den für Hydantoine charakteristischen Banden bei 1720 und 1780 $cm^{-1}$.

Eine Probe der Polyhydantoinlösung wird auf eine Glasplatte aufgestrichen und in jeweils 15 Minuten bei 200°C und 300°C zu einer klaren harten Folie eingebrannt.

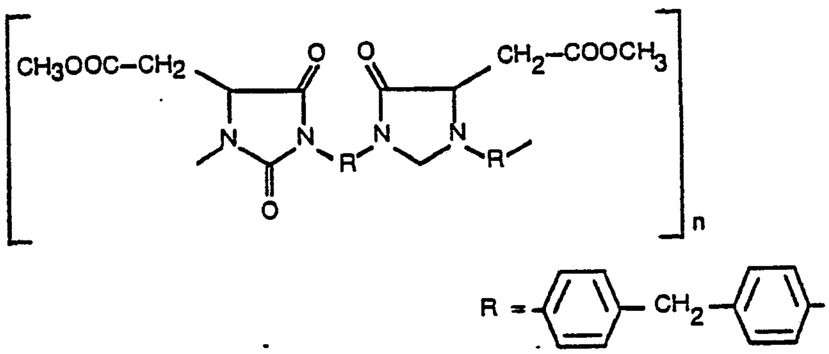

Beispiel 6

In 125 g eines technischen Kresolgemisches werden 45 g 4,4'-Bis-(fumarsäureethylester-amido)-diphenylmethan mit 16,8 g Hexamethylendiisocyanat unter Rühren in 3 Stdn. bei 160°C und 6 Stdn. bei 180°C umgesetzt. Die so hergestellte Lösung eines Poly-hydantoin-(5)-essigsäureethylesters wird bei 200 und 300° auf einem Spiegelblech zu einem klaren harten Lackfilm eingebrannt und zeigt im Relfexionsspektrum die für Hydantoine charakteristischen Banden bei 1710 und 1775 $cm^{-1}$.

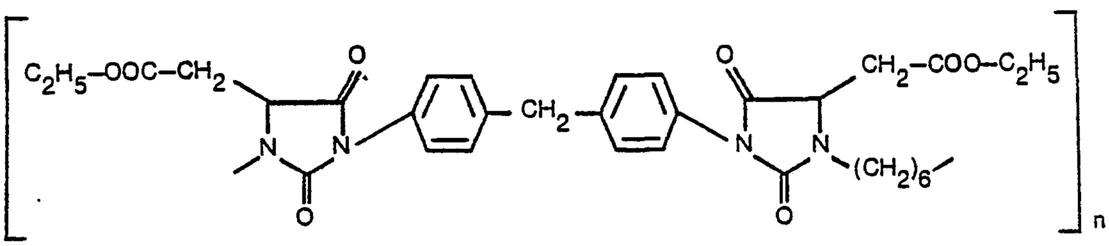

Beispiel 7

18 g 2,4-Bis-(fumarsäuremethylester-amido)-toluol und 9,05 g eines technischen Gemisches aus 80% 2,4- und 20% 2,6-Toluylendiisocyanat werden in 54 g Kresol 2 Stdn. bei 160°C und 4 Stdn. bei 180° gerührt. Man erhält eine Lösung des Poly-hydantoin-(5)-methylesters mit Hydantoin-Banden bei 1730 und 1790 $cm^{-1}$, die bei 200°C und 300°C auf einem Prüfblech zu einem klaren harten Lackfilm eingebrannt wird.

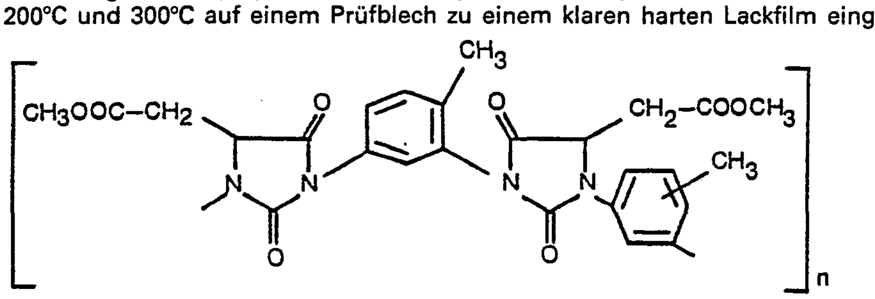

Beispiel 8

6,92 g 2,4-Bis-(maleinsäuremethylester-amido)-toluol und 5,0 g 4,4'-Diisocyanato-diphenylmethan werden unter Stickstoff 2 Stdn. auf 160°C und 2 Stdn auf 180°C erhitzt. Man erhält den Poly-

hydantoin-(5-methylester als hellbraunes Harz mit Hydantoin-Banden im IR-Spektrum bei 1720 und 1780 $cm^{-1}$.

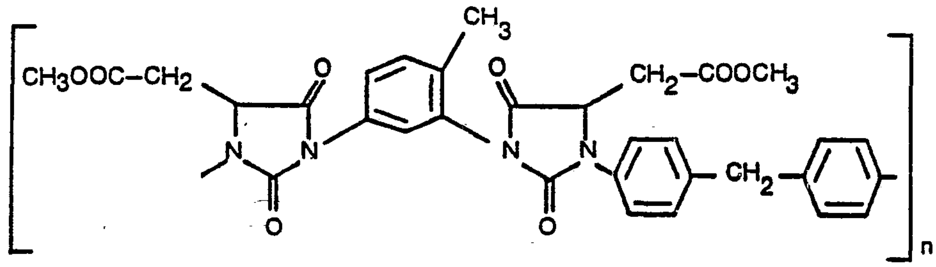

$C_{32}H_{28}N_4O_8$(596)

| | C | H | N |
|---|---|---|---|
| ber. | 64,6 | 4,7 | 9,4% |
| gef. | 64,5 | 5,0 | 9,5% |

Eine Probe des Polyhydantoins wird in Kresol gelöst und bei 200°C und 300°C zu einem klaren elastischen Lackfilm eingebrannt.

**Patentansprüche**

1. Verfahren zur Herstellung von Hydantoinestern, dadurch gekennzeichnet, daß organische Isocyanate mit Ethen-1,2-dicarbonsäureamidestern.

$$(R_4{-}O{-}\overset{\overset{O}{\|}}{C}{-}\overset{\overset{R_1}{|}}{C}{=}\overset{\overset{R_2}{|}}{C}{-}\overset{\overset{O}{\|}}{C}{-}NH{-})_xR_3 \qquad (Ia),$$

$$R_4({-}O{-}\overset{\overset{O}{\|}}{C}{-}\overset{\overset{R_1}{|}}{C}{=}\overset{\overset{R_2}{|}}{C}{-}\overset{\overset{O}{\|}}{C}{-}NH{-}R_3)_y \qquad (Ib)$$

oder

$$\left[{-}R_4{-}O{-}\overset{\overset{O}{\|}}{C}{-}\overset{\overset{R_1}{|}}{C}{=}\overset{\overset{R_2}{|}}{C}{-}\overset{\overset{O}{\|}}{C}{-}NH{-}R_3{-}NH{-}\overset{\overset{O}{\|}}{C}{-}\overset{\overset{R_1}{|}}{C}{=}\overset{\overset{R_2}{|}}{C}{-}\overset{\overset{O}{\|}}{C}{-}O{-}\right]_n \qquad (Ic)$$

in denen

$R^1$, $R^2$, $R^3$ und $R^4$ unabhängig voneiander für einen aliphatischen, cycloaliphatischen, aliphatisch-aromatischen oder aromatischen Rest stehen und

$R^1$, $R^2$ und $R^3$ außerdem noch für Wasserstoff und

$R^1$ und $R^2$ für Halogen,

x und y ganze Zahlen von 1 bis 3 bedeuten und

n eine ganse Zahl von 1 bis 200 bedeutet, bei einer Temperatur von 50 bis 250°C umgesetzt werden.

2. Verfahren nach Anspruch 1, dadurch gekennzeichnet, daß als organische Isocyanate Diisocyanate eingesetzt werden.

**Revendications**

1. Procédé de production d'esters d'hydantoïne, caractérisé en ce qu'on fait réagir des isocyanates organiques avec des esters d'amides d'acides éthène-1,2-dicarboxyliques de formules (Ia—c)

$$(R_4{-}O{-}\overset{\overset{O}{\|}}{C}{-}\overset{\overset{R_1}{|}}{C}{=}\overset{\overset{R_2}{|}}{C}{-}\overset{\overset{O}{\|}}{C}{-}NH{-})_xR_3 \qquad (Ia)$$

$$R_4({-}O{-}\overset{\overset{O}{\|}}{C}{-}\overset{\overset{R_1}{|}}{C}{=}\overset{\overset{R_2}{|}}{C}{-}\overset{\overset{O}{\|}}{C}{-}NH{-}R_3)_y \qquad (Ib)$$

ou

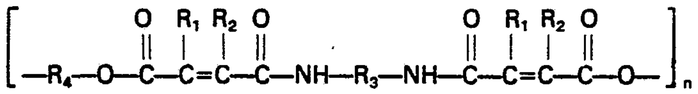

(Ic)

où

. $R^1$, $R^2$, $R^3$ et $R^4$ représentent, indépendamment les uns des autres, un reste aliphatique, cycloaliphatique, aliphato-aromatique ou aromatique et

$R^1$, $R^2$ et $R^3$ représentent, en outre, l'hydrogène et

$R^1$ et $R^2$ représentent, en outre, un halogène,

x et y sont des nombres entiers de 1 à 3 et

n est un nombre entier de 1 à 200, à une température de 50 à 250°C.

2. Procédé suivant la revendication 1, caractérisé en ce qu'un utilise des diisocyanates comme isocyanates organiques.

## Claims

1. Process for the production of hydantoin esters, characterised in that organic isocyanates are reacted with ethene-1,2-dicarboxylic acid amide esters of the formulae (Ia—c)

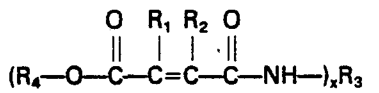

(Ia)

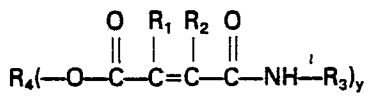

(Ib)

or

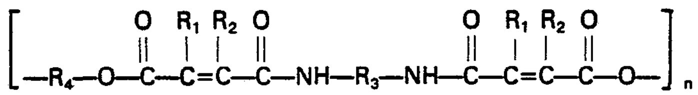

(Ic)

in which

$R^1$, $R^2$, $R^3$ and $R^4$ independently of one another represent an aliphatic, cycloaliphatic, aliphatic-aromatic or aromatic radical and

$R^1$, $R^2$ and $R^3$ also denote hydrogen and

$R^1$ and $R^2$ halogen,

x and y denote integers from 1 to 3 and

n denotes an integer from 1 to 200, at a temperature from 50 to 250°C.

2. Process according to Claim 1, characterised in that diisocyanates are used as organic isocyanates.